# EUROPEAN PATENT SPECIFICATION

(11) **EP 1 790 280 B1**
(45) Date of publication and mention of the grant of the patent: **03.08.2011**
(21) Application number: 06124509.8
(22) Date of filing: 21.11.2006
(51) Int. Cl.: A61B 1/04

(54) **Method of assembling an in-vivo imaging device**
Verfahren für den Zusammenbau einer In-vivo-Bildgebungsvorrichtung
Procédé d'assemblage d'un dispositif d'imagerie in vivo

(30) Priority: 23.11.2005 US 738972 P
(43) Date of publication of application: 30.05.2007
(73) Proprietor: Given Imaging Ltd., 20692 Yoqneam (IL)
(72) Inventor: Gilad, Zvika, 34987, Haifa (IL)
(74) Representative: Harrison Goddard Foote

(56) References cited:
- US-A1- 2002 109 774
- US-A1- 2003 020 810
- US-A1- 2004 027 459
- US-A1- 2005 043 583
- US-A1- 2005 043 586

## Description

### FIELD OF THE INVENTION

The present invention relates to a method of assembling an in-vivo imaging device for capsule endoscopy, as defined in claim 1.

### BACKGROUND OF THE INVENTION

Such in-vivo sensing devices, may be in the form of swallowable or ingestible capsules which may move through a body lumen. The in-vivo sensing device may include, for example, an imaging system for obtaining images from inside the body lumen, such as the gastrointestinal (GI) tract as it moves through it. The imaging system may include, for example, an illumination unit, such as a set of light emitting diodes (LEDs), or other suitable light sources, an imaging sensor and an optical system, which focuses the images onto the imaging sensor. A transmitter and antenna may be included for transmitting the images signals to an external data recorder. A power source, such as one or more batteries, may also be included for powering the various electrical and electronic components. Typically, the imaging system, transmitter, antenna, batteries and other components are assembled in the in-vivo sensing device's housing in a compact and secure manner, which takes into account the cooperation between the electrical and electronic components and the required optical properties of the in-vivo sensing device.
It is known from US 2004/027459 to provide an in-vivo imaging device having a single optical head, and from US 2005/043583 to provide such a device with two optical heads. US 2003/020810 discloses a capsule-type device including a battery.

### SUMMARY OF THE INVENTION

In accordance with the present invention, there is provided a method for assembling an in-vivo imaging device comprising the steps of:
(i) providing two optical heads and a circuit board having two rigid portions connected by a flexible portion;
(ii) attaching the optical heads to the rigid portions;
(iii) providing a first sleeve having two opposing open ends;
(iv) locating said flexible portion in a groove provided within said first sleeve;
(v) folding the circuit board so that the optical heads are positioned over the open ends of said first sleeve;
(vi) placing domes over the optical heads;
(vii) bringing the domes into abutment with the first sleeve so that the first sleeve and the domes form a closed housing enclosing the circuit board and the optical heads; and
(viii) joining the domes to the first sleeve.

In accordance with some embodiments, the comprises the further steps of:
(a) placing at least one battery in a second sleeve having two opposing open ends; and
(b) placing the second sleeve in the first sleeve.

In accordance with some embodiments, the step of placing at least one battery in a second sleeve is performed prior to the step folding the circuit board.

In accordance with some embodiments, at least one battery is placed in the first sleeve.

In accordance with some embodiments, the first sleeve is placed between the two optical heads with the flexible portion passing between the two opposing open ends prior to folding the circuit board; and at least one battery is placed in the first sleeve after positioning one of the optical heads over one of the open ends.

In accordance with some embodiments, the domes are joined to the first sleeve by a process chosen from the following group: gluing, fraction fitting, press fitting, snap fitting, laser welding, laser melting, spin welding, and ultra sonic welding.

### BRIEF DESCRIPTION OF THE DRAWINGS

The present invention will be understood and appreciated more fully from the following detailed description taken in conjunction with the appended drawings in which:

Figs. 1A and 1B schematically illustrate an in vivo imaging device and system according to some embodiments of the present invention;

Fig. 2 schematically illustrates a perspective view of an in vivo imaging device according to some embodiments of the present invention in a body lumen;

Fig. 3 schematically illustrates a longitudinal cross section of an in vivo imaging device according to some embodiments of the present invention;

Figs. 4A and 4B schematically illustrate a top view and a bottom view, respectively, of a circuit board, in accordance with an embodiment of the present invention;

Fig. 5A schematically illustrates a connecting sleeve, according to some embodiments of the present invention;

Fig. 5B schematically illustrates a side view, of a battery contact, in accordance with some embodiments of the present invention;

Fig. 6A is a schematic flow-chart of a method of assembling an in vivo imaging device, in accordance with some embodiments of the invention;

Figs. 6B -6F schematically illustrate a method of assembling an in vivo imaging device, in accordance with some embodiments of the invention;

Fig. 6G is a schematic flow-chart of a method of assembling an in vivo imaging device, in accordance with some embodiments of the invention;

Fig. 7A is a schematic flow-chart of another method of assembling an in vivo imaging device, in accordance with some embodiments of the present invention;

Fig. 7B schematically illustrates a method of assembling an in vivo imaging device, in accordance with some embodiments of the present invention; and

Fig. 7C schematically illustrates a perspective view of the in vivo imaging device shown in Fig. 7B in an assembled state.

It will be appreciated that for simplicity and clarity of illustration, elements shown in the figures have not necessarily been drawn to scale. For example, the dimensions of some of the elements may be exaggerated relative to other elements for clarity.
Not all figures show all steps of the method in claim 1.

### DETAILED DESCRIPTION OF THE INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

It is noted that some embodiments of the present invention may be directed to an autonomous, typically ingestible in-vivo device. Other embodiments need not be ingestible. Devices or systems according to embodiments of the present invention may be similar to embodiments described in International Application WO 01/65995 and/or in U.S. Patent No. 5,604,531, each of which are assigned to the common assignee of the present invention. Furthermore, a receiving and/or display system suitable for use with embodiments of the present invention may also be similar to embodiments described in WO 01/65995 and/or in U.S. Patent Number 5,604,531. Devices and systems as described herein may have other configurations and other sets of components.

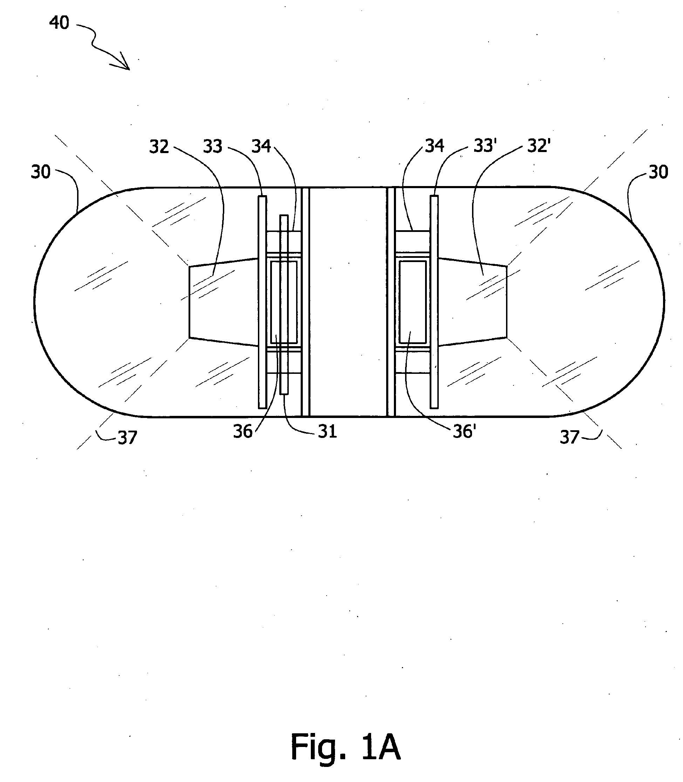

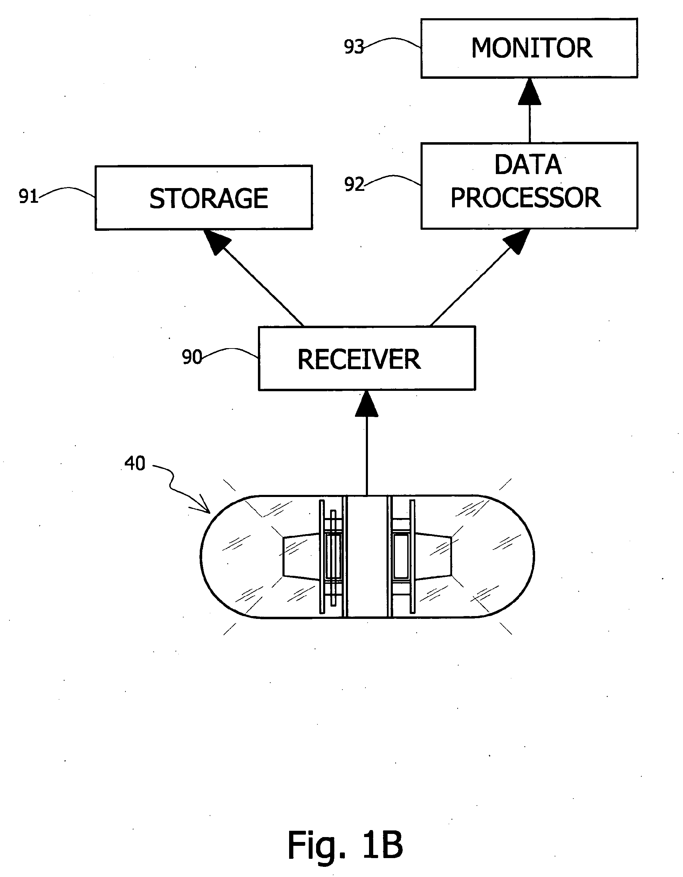

Reference is made to Fig. 1, which shows a schematic diagram of an embodiment of an in-vivo imaging device 40 and transmitter/receiver 31 in accordance with an embodiment of the invention. In one embodiment, the system may include a device 40 having an imager 36 and/or 36' (such as for example a CMOS, a CCD, etc.), an optical system which may include lens holder 32 and/or 32', lenses and other optical elements and illumination sources 34 such as one or more LEDs (Light Emitting Diode), and/or OLEDs (Organic LED) or other suitable illumination sources. According to one embodiment the imager, optical system and light source are positioned behind a viewing window 30. Viewing window 30 may be a transparent elongated dome. The device may include a power source such as silver oxide batteries, lithium batteries, other suitable electrochemical cells having a high energy density, or the like. Other power sources may be used. For example, instead of internal power source or in addition to it, an external power source may be used to transmit power to device 40. In some embodiments, an additional sensor may be included in the device, for example, pH, temperature, pressure or other physiological parameter sensors. Other components or sensors may also be included. A processor may be included in the device which may be for example capable of processing signals that are received by device 40 into for example command or control signals that may control, activate, deactivate or otherwise alter an operative state of components that may be included in device 40. The transceiver 31 may be a transmitter or a receiver or both that may be capable of receiving wireless signals and transmitting wireless signals; in some embodiments only transmission (for example, transmission of image data from imagers 36 and/or 36') may occur. Transceiver 31 may also have other functions. In some embodiments, transceiver 31 and the processor may be or may be included in a single integrated circuit. Device 40 may include antenna that may be operably attached to transceiver 31. In some embodiments, the antenna may be used for, or in the performance of, both the receipt and transmission of wireless signals by transceiver 31. In other embodiments there may be more than one antenna. In some embodiments, device 40 may transmit but not receive signals. An additional sensor or other components need not necessarily be included.

According to one embodiment of the present invention, device 40 may include two optical units. Each optical unit may include, for example, the transparent elongated dome 30 behind which are situated illumination sources 34, lens holders 32, 32' and imager 36, 36'. According to some embodiments of the present invention, device 40 is capable of simultaneously obtaining images of the body lumen, for example, the GI tract, from two ends of the device. For example, according to one embodiment of the present invention, device 40 may be a cylindrical capsule having a front end and a rear end, which is capable of passing the entire GI tract. The front and rear ends may define a longitudinal direction and a longitudinal axis of the device 40. The lens holders 32, 32' and imagers 36, 36' may be located along the longitudinal axis. The imagers 36, 36' may be perpendicular to the longitudinal axis. The system in a cylindrical capsule can image the GI tract in the front and in the rear of the capsule. The images may be transmitted simultaneously or serially and may be displayed separately or as a single combined image.

When used herein, terms like top, bottom, front, rear, over, above, etc., are considered relative terms descriptive of, for example, when the imaging device 40 is in a specific orientation relative to the viewer or the relative position of components of the device.

According to some embodiments of the present invention, the device 40 may include one or more light blockers such as light blockers 33 and 33' which may include a suitable structure to reduce backscatter. In some embodiments, the light blocker may be formed and/or shaped such that it blocks stray light from reaching and/or flooding the imagers, such as imager 36 and imager 36'.

According to some embodiments the optical system in the device 40 may enable a wide field of view 37.

Fig. 1B is a schematic illustration of an in-vivo imaging system in accordance with some embodiments of the present invention. External to device 40 may be the receiver 90 and possibly a transmitter. Receiver 90 and a possible transmitter (typically including or associated with an antenna or antenna array) may be housed or included in the same housing or unit, or may be housed in one or more separate units. For example, a transmitter and receiver may be housed in a portable unit that may be carried or worn by a patient and/or may be integrated into a transceiver.

Receiver 90 may be connected to and/or in electrical communication with a processor 92 which may process, for example, data signals such as, for example, sensory or image data signals that are received from device 40 and/or control data received from device 40. In some embodiments, receiver 90 may be operably connected to a monitor/display 93 and/or a storage system 91 that may display and/or store the image or other sensory data collected and transmitted by device 40. Processor 92 may analyze data received by receiver 90 and may be in communication with storage system 91, transferring image data (which may be stored and transferred as for example frame data) or other data to and from storage system 91. Processor 92 may also provide the analyzed data to display 93 where a user may view the images. Display 93 may present or display the data such as, for example, image frame data or video data of, for example, the gastro-intestinal (GI) tract or other body lumen. In one embodiment, processor 92 may be configured for real time processing and/or for post processing to be performed. Other monitoring and receiving systems may be used.

A transmitter may typically be connected to and/or in electrical communication with processor 92. Processor 92 may function, at least partially as a controller and/or include, for example, a controller to process, for example, control commands to device 40 via the transmitter. In other embodiments of the present invention, signals other than control commands may be processed by processor 92 with, for example, the controller and transmitted via the transmitter. In yet other embodiments, the controller and processor may be separate units that may be in electrical communication with each other. In some embodiments of the present invention, control commands generated, for example, by the controller may be based on data received by the receiver 90 and processed by processor 92. In other embodiments, control commands generated, by the controller may be based on, user input data, for example, a patient or external operator may for example, initiate the transmission of a wireless signal and/or command from, for example, the transmitter to transceiver 31. In yet other embodiments, control commands may be based on both user input data and data receiver and/or processed by processor 92.

In some embodiments, transceiver 31 may be a half duplex transceiver where the transceiver 31 alternates from transmitting to receiving, e.g. via time division multiple access (TDMA). Typically, the transmission rate to the external receiver 90 may be significantly higher than the transmission rate from external transmitter to the transceiver 31. For example, device 40 may transmit, e.g. image frame data to external receiver 90 at a rate of 1-10 Mbits/s, e.g. 2.7 Mbits/s, while the external transmitter may transmit control commands to the transceiver 31 that may be at rate of 10-30 Kbits/sec.

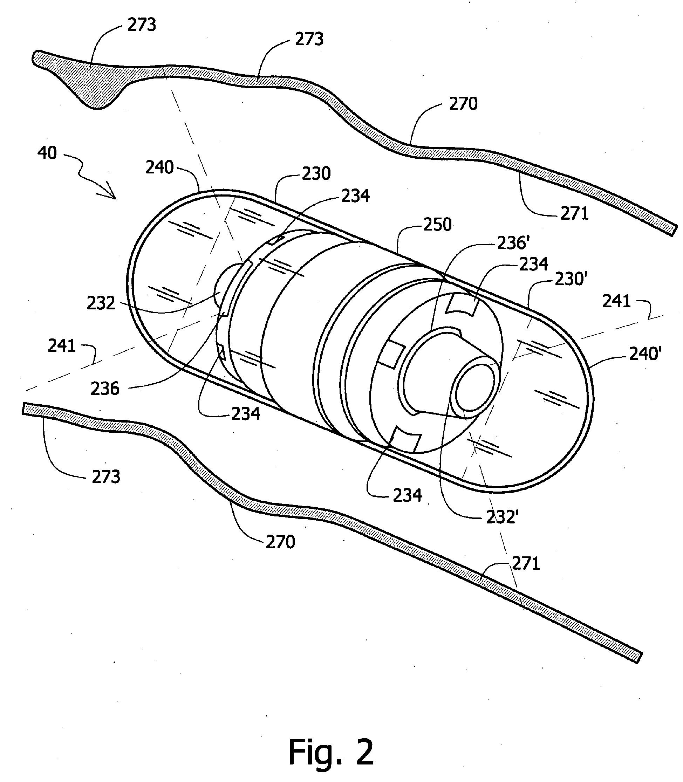

Fig. 2 is a schematic illustration of in-vivo imaging device 40 in accordance with some embodiments of the present invention. According to one embodiment of the present invention, device 40 may be partially or entirely transparent. For example, device 40 may include areas, such as a front and rear transparent optical domes 230 and 230', which may allow components inside device 40 to have an un-obstructed field-of-view of the environment external to device 40. Other shaped transparent areas may be used. The front and rear transparent optical domes 230 and 230' may define a longitudinal direction and a longitudinal axis of the device 40.

According to one embodiment of the present invention, each of the transparent domes 230 and 230' may, respectively, include viewing windows 240 and 240'. According to some embodiments of the present invention the viewing windows 240 and 240' may for example be transparent to the light emitted by illumination sources 234 that is reflected back off of, for example, an endo-luminal wall to device 40. According to some embodiments of the present invention, the transparent domes 230 and 230' may be configured such that an appropriate field of view and/or field of illumination of the body lumen walls may be achieved with a reduced risk of stray light or backscatter from illumination sources 234 onto imagers 236 and 236'. The imagers 236, 236' may be located along the longitudinal axis and may be perpendicular thereto. According to some embodiments of the present invention the two viewing windows 240 and 240' may be configured such that a field of view 241 in the range of between 80 - 150 degrees is enabled; other suitable fields of view may be used. According to one embodiment of the present invention the effective focal distance (also referred to as the effective focal length), of the device 40 may typically be between 0 to 40 mm; however, other suitable distances may be used.

In one embodiment, as device 40 traverses body lumen 270, device 40 may capture images substantially simultaneously of one or more areas of body lumen 270, such as locations 271 and 273. According to some embodiments of the present invention illumination sources 234 may illuminate locations 271 and 273 of body lumen 270. The light from illuminated locations 271 and 273 may be reflected, focused and/or transferred using the optical system which may include lens holders 232 and 232', and received by imagers 236 and 236', which may thereby capture an image of locations 271 and 273. The lens holders 232, 232' may be located along the longitudinal axis.

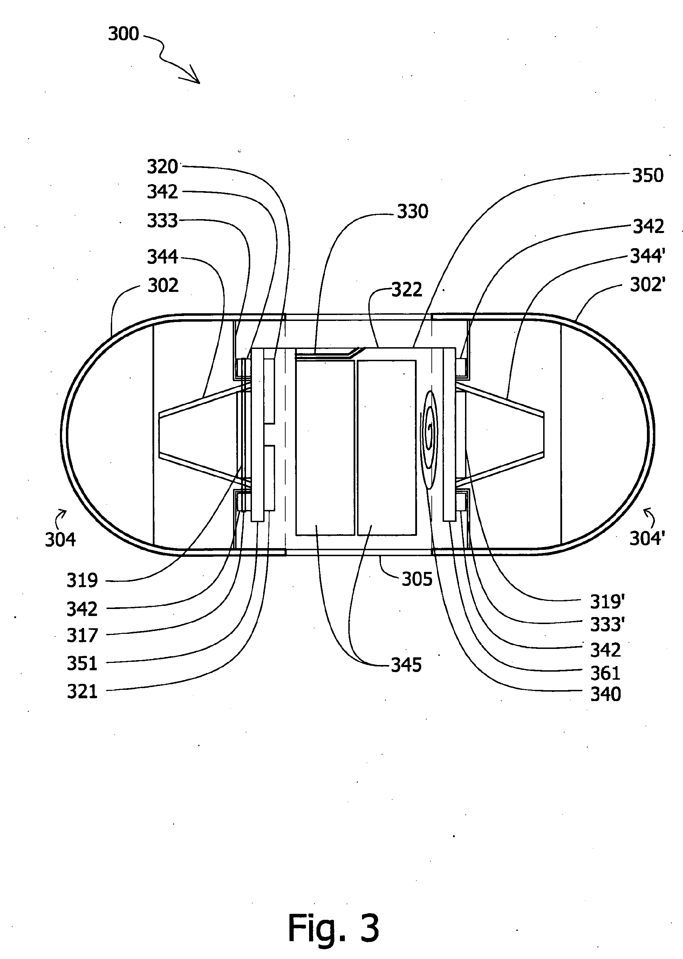

Reference is made to Fig. 3, which shows a schematic representation of a longitudinal cross-section of a device 300 according to embodiments of the present invention. The device 300 may include two optical domes 302 and 302'. According to one embodiment of the present invention each optical dome 302 and 302' may be an integral part of two elongated ends of a capsule, such as a transparent front end 304 and a transparent rear end 304'. According to one embodiment of the present invention the front and rear ends 304 and 304' may be attached to a connecting sleeve, for example an opaque sleeve 305 having two opposing open ends. According to some embodiments of the present invention behind the transparent ends 304 and 304' may be, respectively, situated for example illumination sources 342, lens holder 344 and 344', imagers 319 and 319' a transmitter/receiver such as an ASIC 320 and a switch 321 such as a MEMS switch or a reed switch RI-80 SMD. The lens holders 344, 344' contain various optical components (not shown), such as optical lenses, for focusing light on the imagers 319, 319'. Each lens holder 344, 344' along with its associated optical components is referred to herein as an optical head. The device 300 may further include one or more power sources 345, such as E370 or E399 or GP370 batteries, which may provide power to the entirety of electrical elements of the device, and an antenna 317 for transmitting and/or receiving, for example, image signals from the imagers 319 and 319'. According to some embodiments of the present invention, device 300 is capable of simultaneously obtaining images of the body lumen, for example, the GI tract, from two ends of the device. For example, according to one embodiment of the present invention device 300 may be a floatable capsule having a front end and a rear end, which is capable of passing the entire GI tract.

According to one embodiment of the present invention the device 300 may include two battery contacts, such as battery contact 330 which may be located at the sides of the batteries 345, and battery contact 340 which may be located beneath the batteries 345.

The various components of the device 300 are disposed on a circuit board 350 including rigid and flexible portions; preferably the components are arranged in a stacked vertical fashion. For example, rigid portion 351 of the circuit board 350 may hold an imager 319, an antenna 317, a lens holder 344 and a light blocker 333, while rigid portion 361 may hold a lens holder 344', an imager 319' and a light blocker 333'. According to one embodiment of the present invention, the other side of the rigid portion 351 may include, for example, a transmitter/receiver 320 and a switch 321, while the other side of rigid portion 361 may hold a battery contact 340 for battery or power source(s) 345. According to one embodiment of the present invention, rigid portions 351 and 361 of the circuit board 320 may include, for example, an illumination source, such as one or more LEDs 342 or other illumination sources. According to some embodiments of the present invention, each rigid portion of the circuit board may be connected to another rigid portion of the circuit board by a flexible connector portion 322 of the circuit board 350. According to one embodiment of the present invention, each rigid portion of the circuit board may include two rigid sections; sandwiched between the rigid sections is a flexible connector portion of the circuit board for connecting the rigid boards. In alternate embodiments, other arrangements of components may be placed on a circuit board having rigid portions connected by flexible portions.

Arrangements of components as described above may be included in other capsule shaped devices, for example, a device having only one transparent dome and one imager for imaging from only one end of the device.

According to one embodiment components may be arranged in an in vivo autonomous imaging device on an array of chips using flip chip bonding.

In alternate embodiments, a circuit board having rigid portions and flexible portions may be used to arrange and hold components in other in vivo sensing devices, such as a swallowable capsule measuring pH, temperature or pressure, or in a swallowable imaging capsule having components other than those described above. Such circuit boards may be similar to embodiments described in US application number 10/879,054 entitled IN VIVO DEVICE WITH FLEXIBLE CIRCUIT BOARD AND METHOD FOR ASSEMBLY THEREOF, and US application number 60/298,387 entitled IN VIVO SENSING DEVICE WITH A CIRCUIT BOARD HAVING RIGID SECTIONS AND FLEXIBLE SECTIONS.

According to some embodiments of the present invention, one or more components of device 300, for example the lens holders 344 and 344', the imagers 319 and 319', the transmitter 320 and the switch 321 may be packaged and may be further attached and/or interconnected for example, to the circuit board 350 using three dimensions (3D) chip scale packaging techniques. For example, according to one embodiment of the present invention, the lens holder 344, the imager 319, the transmitter 320 and the circuit board 320 may be interconnected to one another by using, for example a bonding layer such as a Solder Bumps layer.

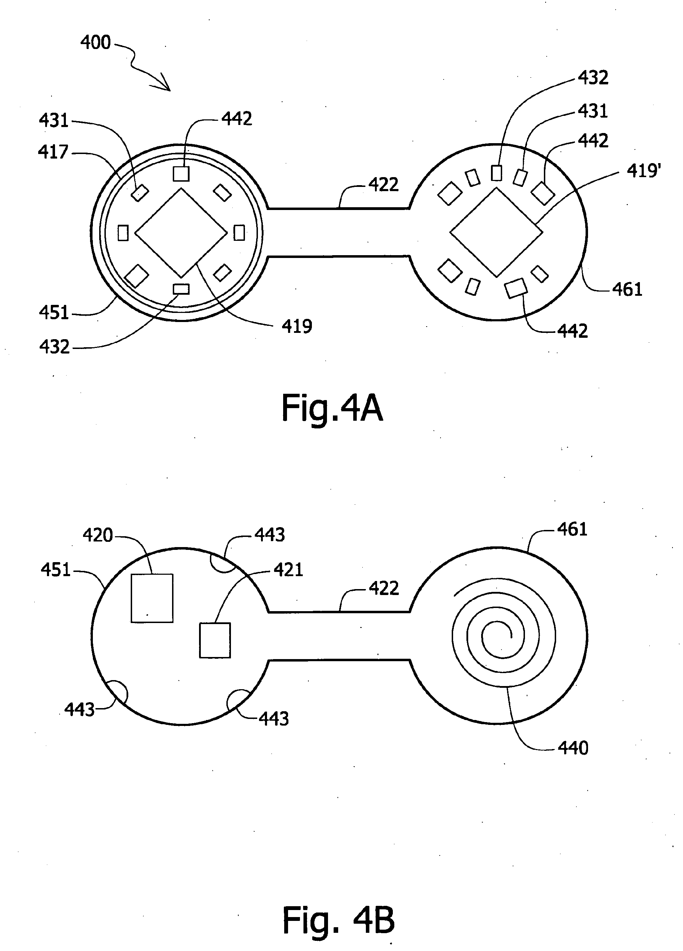

Figs. 4A and 4B schematically illustrate a top view and a bottom view, respectively, of a circuit board 400 in accordance with some embodiments of the invention. In some embodiments, circuit board 400 may be an example of circuit board 350 of FIG. 3. In some embodiments, circuit board 400 may be used in conjunction with device 40 of FIG. 1, or with other suitable devices and systems for in vivo sensing or in vivo imaging, for example, in a capsule having only one transparent dome and one imager for imaging from only one end of the capsule.

According to some embodiments of the present invention, circuit board 400 may include, for example, one or more rigid portions and one or more flexible portions. For example, circuit board 400 may include rigid portions 451 and 461, which may be interconnected using flexible portion 422. Although two rigid portions and one flexible portion are shown, embodiments of the present invention are not limited in this regard, and may include other numbers, orders or combinations of rigid portions and/or flexible portions.

In some embodiments, rigid portion 451 and/or rigid portion 461 may include, for example, one or more illumination sources 442 such as LEDs and/or OLEDs, and optionally one or more resistors 431 and capacitors 432 to regulate or control the power provided to illumination sources 442. Although two rigid portions 451 and 461 having illumination sources 442 are shown, embodiments of the invention are not limited in this regard; for example, in one embodiment, circuit board 400 may include rigid portion 451 and may not include rigid portion 461.

In some embodiments, rigid portion 451 may include a first imager 419 an antenna 417 a transmitter/receiver such as an ASIC 420, a switch 421 and one or more battery contact pads 443 for connecting the electrical components of the in-vivo device 300 to the battery 345.

In some embodiments, rigid portion 461 may include a battery holder 440, e.g., a spring able to hold a battery, such as battery 345, or other power source in place. According to some embodiments the present invention, rigid portion 461 may optionally include a second imager 419'. Although two imagers 419 and 419' are shown, embodiments of the invention are not limited in this regard; for example, in one embodiment, circuit board 400 may include one imager, or another suitable number of imagers.

According to some embodiments of the present invention, the one or more flexible portions of circuit board 400 may allow bending, folding, twisting or positioning of circuit board 400 into certain shapes. For example, circuit board 400 may have a "C" shape as shown in FIG. 3 or other suitable shapes.

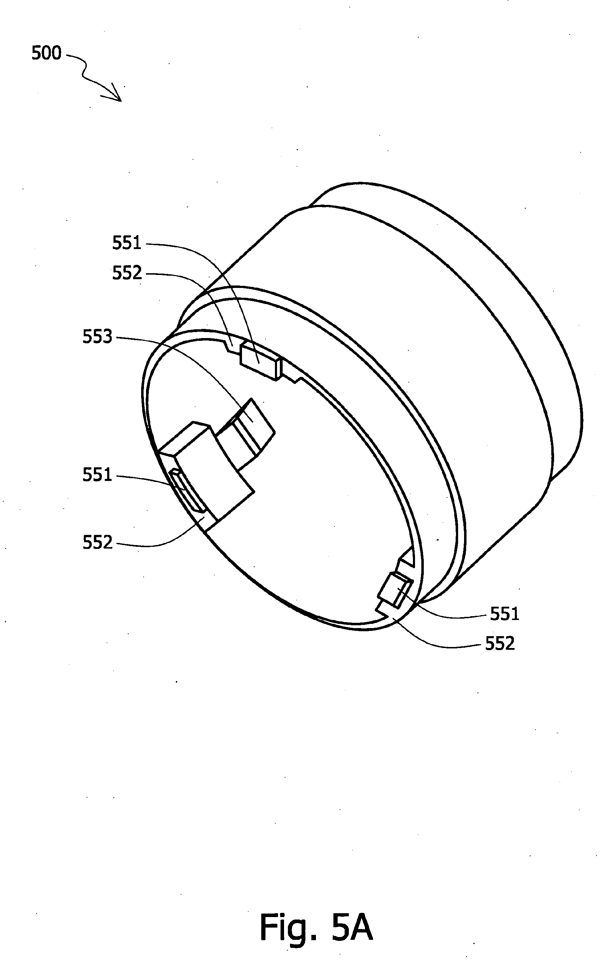

Reference is now made to Fig. 5A which schematically illustrates a connecting sleeve 500 according to some embodiments of the present invention. In some embodiments, connecting sleeve 500 may be used in conjunction with device 40 of FIG. 1, or with other suitable devices and systems for in vivo sensing or in vivo imaging.

According to one embodiment of the present invention the connecting sleeve 500 may include for example three battery contacts 551. According to one embodiment of the present invention the battery connects 551 may be placed, for example in the inside section of the connecting sleeve 500. The battery contacts 551 may be reed shaped and may be inserted, for example on three protrusions 552 from the sleeve inner wall. According to one embodiment of the present invention, the three protrusions 552 may be integral to the connecting sleeve 500 inner wall.

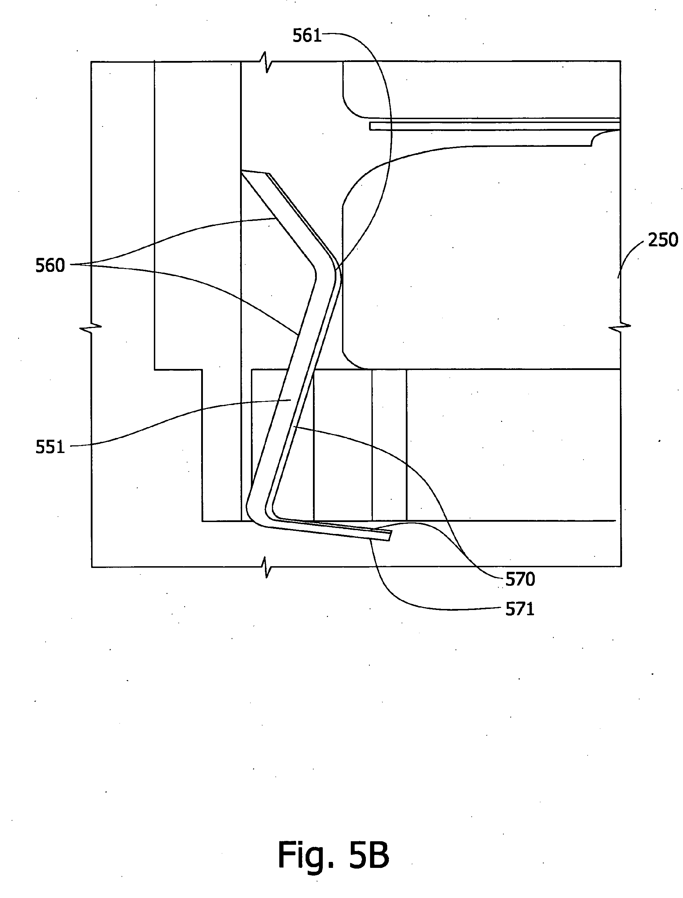

Fig. 5B schematically illustrates a side view, of a battery contact, for example the battery contact 551, in accordance with some embodiments of the present invention. According to one embodiment of the present invention one edge of the battery contact 551, for example edge 560, may have a shape of, for example, a plate, and may include a connection point 561 which may be used as a connection point between the battery 250 and the battery contact 551. According to one embodiment of the present invention, the other edge of the battery contact, for example edge 570 may be shaped for example as a boomerang, and may include a connection point (e.g. 571) between the battery contact 551 and the battery contact pads 443 (shown in Fig. 4B).

A battery contact such as described above may be used in other in vivo imaging device, such as in a capsule having only one transparent dome and one imager for imaging from only one end of the capsule. The battery contact 551 is only one illustrative example of a battery contact that may be used with the present invention. Other types of battery contacts may also be used with the present invention.

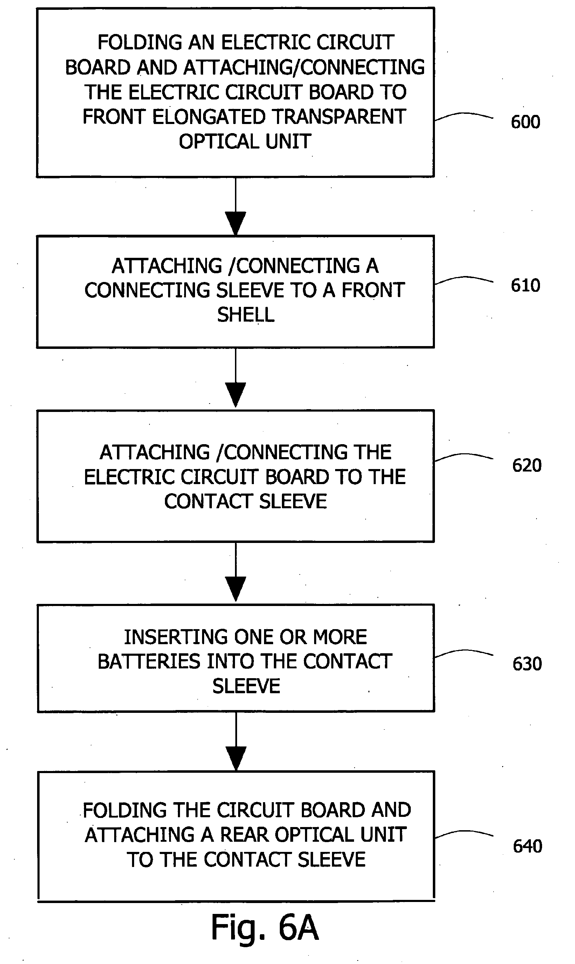

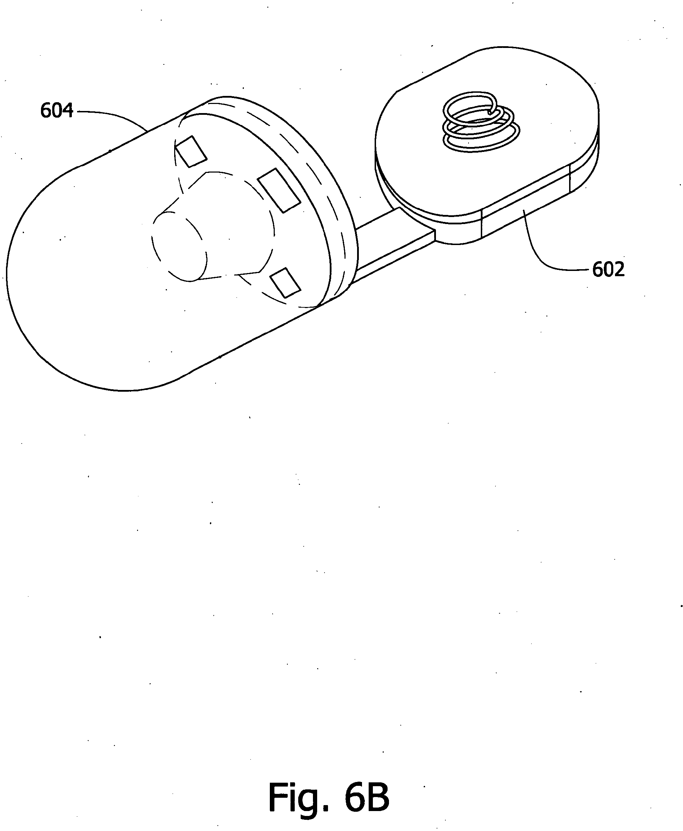

FIG. 6A is a schematic flow-chart of a method of assembling an in vivo imaging device, such as device 300 of Fig. 3, in accordance with some embodiments of the invention. As indicated at box 600, the method may optionally include folding an electric circuit board, such as a rigid-flex circuit board 602, and attaching or connecting the electric circuit board 602 to an optical unit, for example a front elongated transparent optical unit 604, as shown in Fig. 6B.

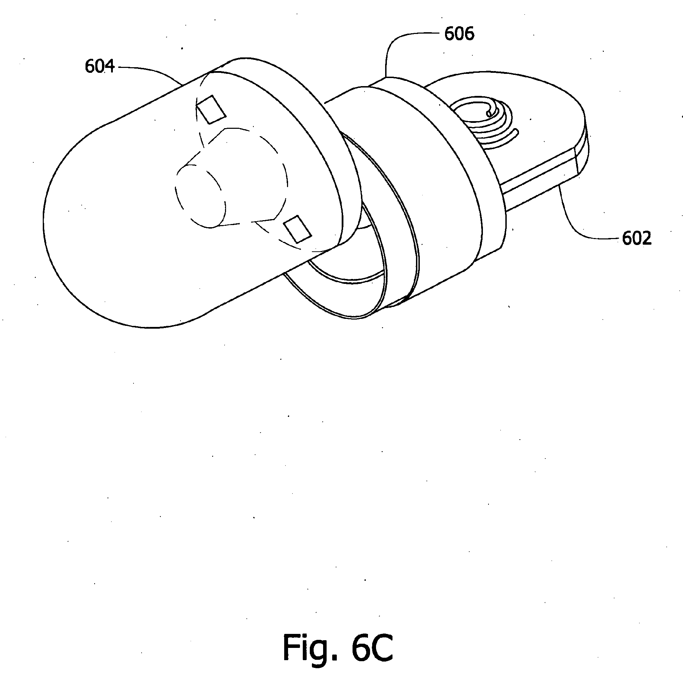

As indicated at box 610, the method may optionally include attaching or connecting a connecting sleeve, such as, according to one embodiment, a nontransparent connecting sleeve 606 to the front elongated transparent optical unit 604 as shown in Fig. 6C.

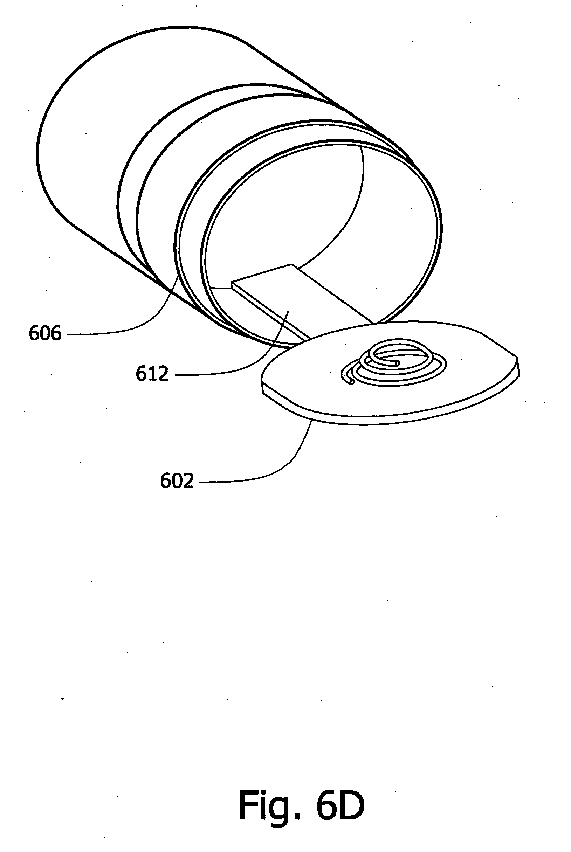

As indicated at box 620, the method includes attaching or connecting the electric circuit board to the sleeve 606 as shown in Fig 6D. Specifically, a flexible portion 612 of the electric circuit board 602 is located in a groove (not seen) of the connecting sleeve 606.

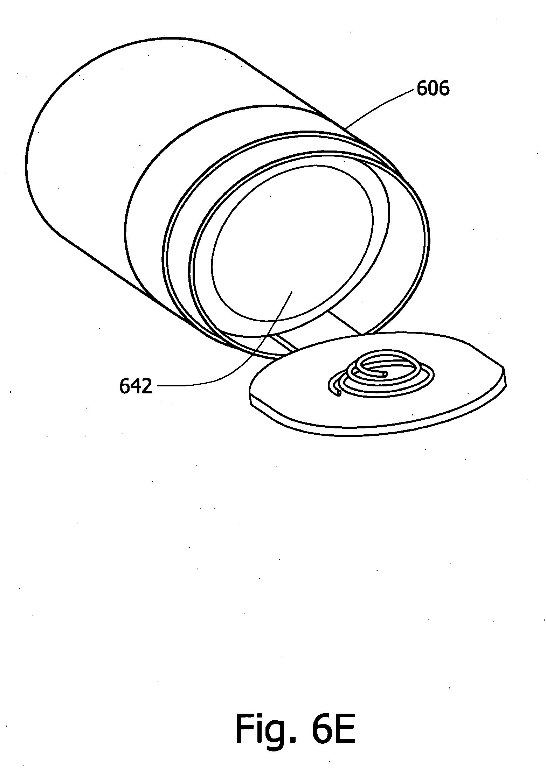

As indicated at box 630, the method may optionally include inserting one or more batteries, such as batteries 642 into the connecting sleeve 606, as shown in Fig. 6E.

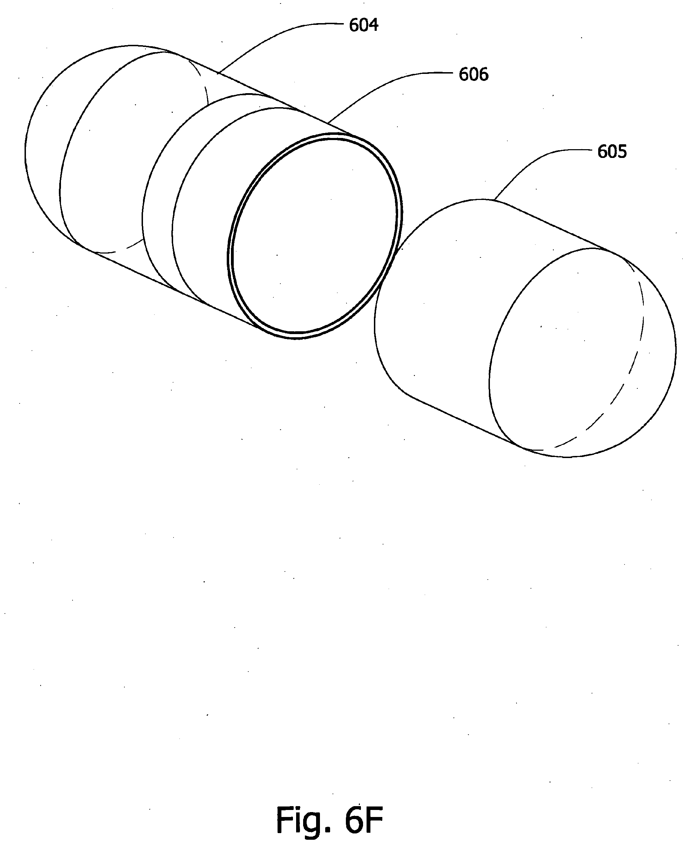

As indicated at box 640, according to one embodiment of the present invention, the method may optionally include, folding the circuit board 602 and attaching a rear optical unit to the connecting sleeve 606. For example, according to one embodiment of the present invention, as shown Fig. 6F, a transparent optical rear unit 605 may be attached or connected to the connecting sleeve 606.

The method of assembling an in vivo imaging device, such as device 300 of Fig. 3, as described above with respect to Fig. 6A, may be carried out in any desired order and is not restricted to the order of the steps as shown in Fig. 6A.

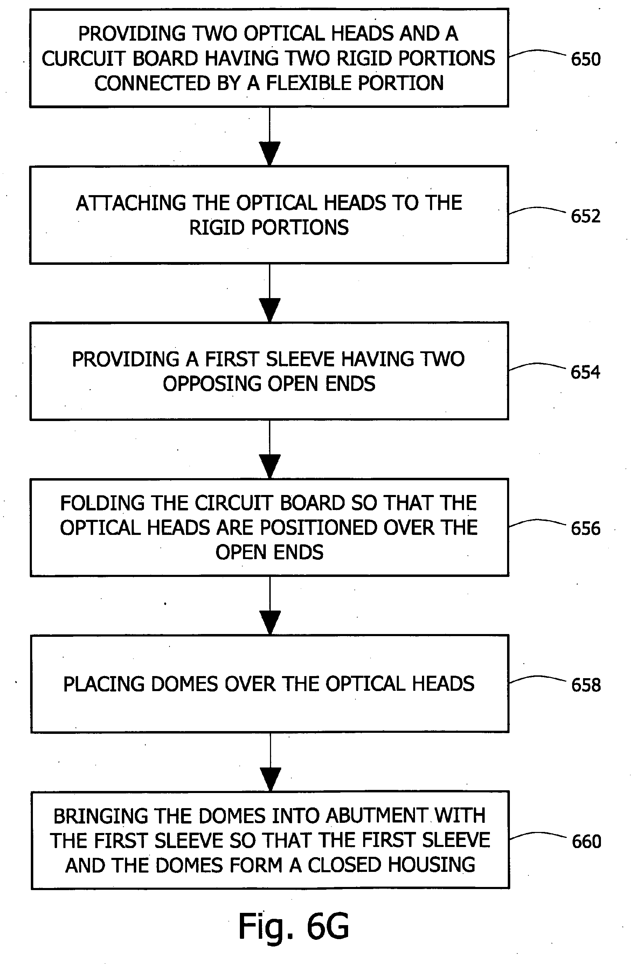

Fig. 6G is a schematic flow-chart of another method of assembling an in vivo imaging device, such as device 300 of Fig. 3, in accordance with some embodiments of the invention. As indicated in box 650, the method may optionally include the step of providing two optical heads (as mentioned above, an optical head is referred to herein as lens holder 344, 344' along with its associated optical components). As indicated in box 652, the method may optionally include the step of attaching the optical heads to the circuit board rigid portions 602. As indicated in box 654, the method may optionally include the step of providing the connecting sleeve 606. The connecting sleeve 606 is generally cylindrical in form, having two opposing open ends. As indicated in box 656, the method may optionally include the step of folding the circuit board so that the optical heads are positioned over the open ends. As indicated in box 658, the method may optionally include the step of placing domes 302, 302' (or, equivalently, elongated ends 304, 304' of the device 300) over the optical heads. As indicated in box 660, the method may optionally include the step of bringing the domes 302, 302' (or, equivalently, elongated ends 304, 304' of the device 300) into abutment with the connecting sleeve 606 so that the connecting sleeve 606 and the domes 302, 302' form a closed housing. The closed housing defines the boundary surface of the in-vivo device 300.

In accordance with some embodiments, the method may comprise the optional step of placing at least one battery 345 in a holding sleeve (not shown) prior to being placed in the connecting sleeve 606. The holding sleeve may aid in holding a number of batteries together as a single battery pack. The holding sleeve may have two opposing open ends. In accordance with some embodiments step of placing at least one battery 345 in the holding sleeve is performed prior to the step of folding the circuit board so that the optical heads are positioned over the open ends.

In accordance with some embodiments, the method may comprise the optional step of placing the at least one battery 345 in placing at least one battery in the connecting sleeve 606.

In accordance with some embodiments, the method may comprise placing the connecting sleeve 606 between the two optical heads with the flexible portion 612 passing between the two opposing open ends prior to the step of folding the circuit board; and the at least one battery is placed in the connecting sleeve 606 after positioning one of the optical heads over one of the open ends of the connecting sleeve 606.

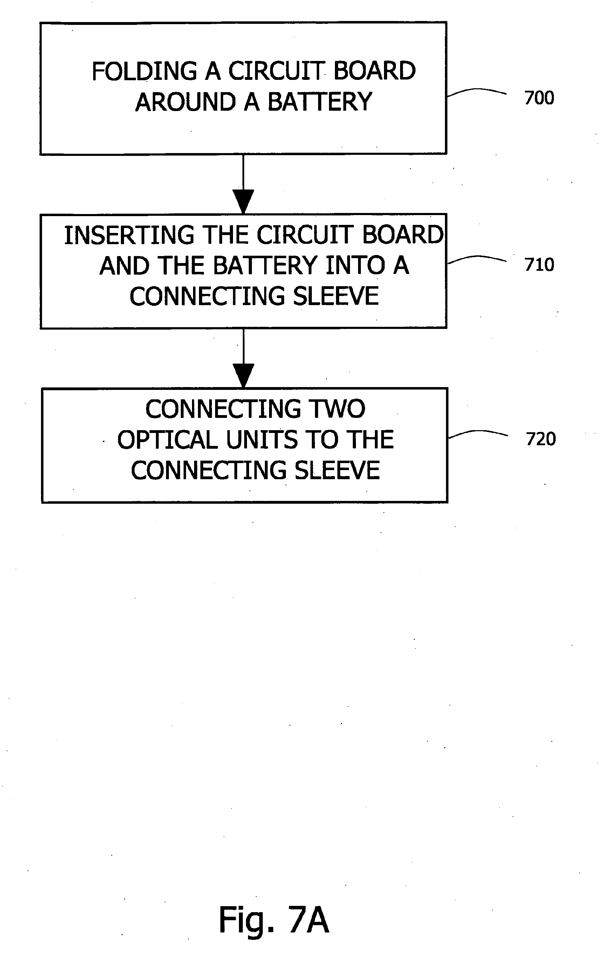

FIG. 7A is a schematic flow-chart of another method of assembling an in vivo imaging device, such as device 300 of Fig. 3, in accordance with some embodiments of the invention. As indicated at box 700, the method may optionally include folding an electric circuit board. For example folding a rigid -flex circuit board around a battery.

As indicated at box 710, according to one embodiment of the present invention, the method may optionally include inserting the rigid-flex circuit board and the battery to a connecting sleeve for example to a nontransparent connecting sleeve.

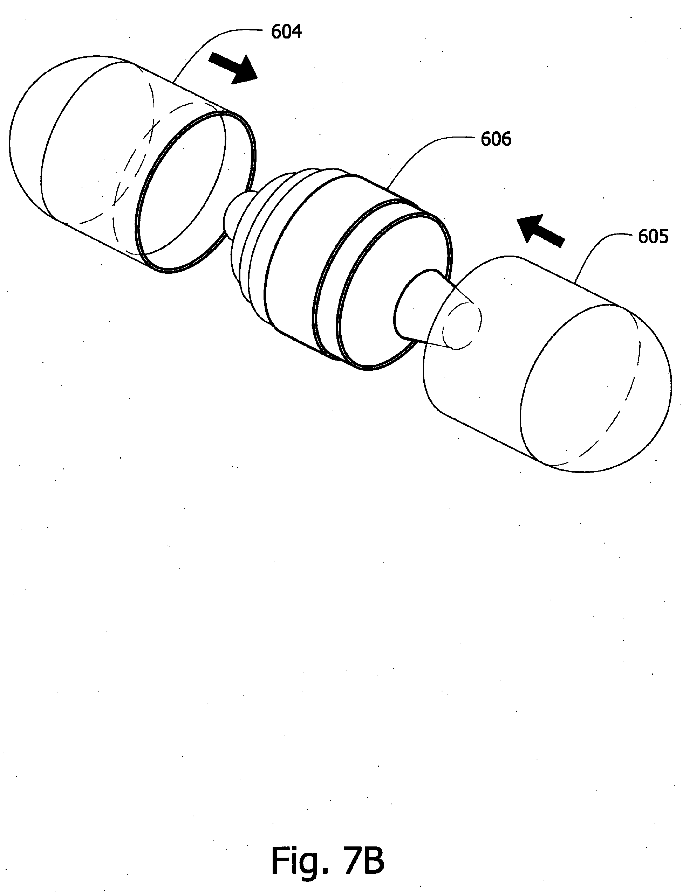

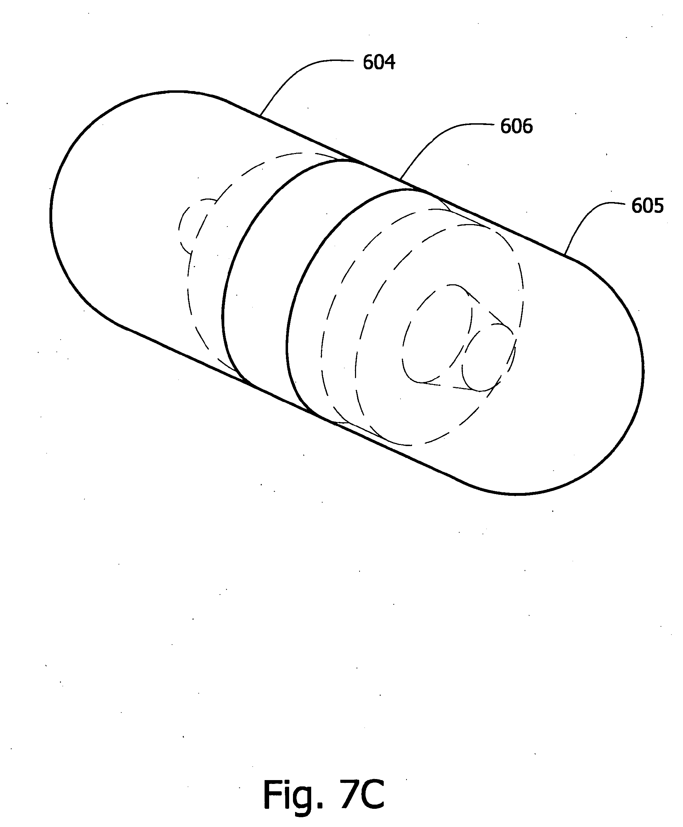

As indicated at box 720, the method may optionally include connecting two optical units to the connecting sleeve 606, for example connecting two transparent elongated front and rear optical units 604 and 605, to the nontransparent connecting sleeve 606 as shown in Figs. 7B and 7C.

According to some embodiment of the present invention, the in vivo imaging device components, such as the front and rear transparent optical units 604 and 605 and the connecting sleeve 606 may be joined together by using one or more of the following methods: fraction fitting, press fitting, snap fitting, laser welding, laser melting, spin welding, and ultra sonic welding.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the scope of the claims.
Throughout the description and claims of this specification, the words "comprise" and "contain" and variations of the words, for example "comprising" and "comprises", means "including but not limited to", and is not intended to (and does not) exclude other moieties, additives, components, integers or steps.
Throughout the description and claims of this specification, the singular encompasses the plural unless the context otherwise requires. In particular, where the indefinite article is used, the specification is to be understood as contemplating plurality as well as singularity, unless the context requires otherwise.
Features, integers, characteristics, compounds, chemical moieties or groups described in conjunction with a particular aspect, embodiment or example of the invention are to be understood to be applicable to any other aspect, embodiment or example described herein unless incompatible therewith.

## Claims

1. A method for assembling an in-vivo imaging device (40) comprising the steps of:
(i) providing two optical heads and a circuit board (350) having two rigid portions (351, 361) connected by a flexible portion (322);
(ii) attaching the optical heads to the rigid portions (351, 361);
(iii) providing a first sleeve (305) having two opposing open ends;
(iv) locating said flexible portion (322) in a groove provided within said first sleeve (305);
(v) folding the circuit board (350) so that the optical heads are positioned over the open ends of said first sleeve (305);
(vi) placing domes (302, 302') over the optical heads;
(vii) bringing the domes (302, 302') into abutment with the first sleeve (305) so that the first sleeve (305) and the domes (302, 302') form a closed housing enclosing the circuit board (350) and the optical heads; and
(viii) joining the domes (302, 302') to the first sleeve (305).

2. The method according to claim 1, comprising the further steps of:
(a) placing at least one battery (345) in a second sleeve having two opposing open ends; and
(b) placing the second sleeve in the first sleeve (305).

3. The method according to claim 2, wherein steps (a) and (b) are performed prior to step (v).

4. The method according to any preceding claim, comprising the further step of placing at least one battery (345) in the first sleeve (305), wherein said further step is performed prior to step (v).

5. The method according to any one of claims 1 to 3, wherein at least one battery (345) is placed in the first sleeve (305) after positioning one of the optical heads over one of the open ends.

6. The method according to any preceding claim, wherein the domes (302, 302') are joined to the first sleeve (305) by a process chosen from the following group: gluing, friction fitting, press fitting, snap fitting, laser welding, laser melting, spin welding, and ultra sonic welding.

7. The method according to any preceding claim, further comprising a step of inserting at least one battery contact (551) in at least one protrusion (552) provided within said first sleeve (305 to 500).

8. The method according to any preceding claim, wherein the step of locating further comprises passing the flexible portion (322) longitudinally between the opposing open ends of said first sleeve (305).

## Patentansprüche

1. Verfahren zum Zusammenbauen einer In-Vivo-Bildaufahmevorrichtung (40), mit den folgenden Schritten:
(i) Bereitstellen zweier optischer Köpfe und einer Leiterplatte (350), die zwei starre Abschnitte (351, 361) aufweist, die mit einem flexiblen Abschnitt (322) verbunden sind;
(ii) Anbringen der optischen Köpfe an den starren Abschnitten (351, 361);
(iii) Bereitstellen einer ersten Hülse (305), die zwei entgegengesetzte offene Enden aufweist;
(iv) Anordnen des flexiblen Abschnitts (322) in einer in der ersten Hülse (305) bereitgestellten Nut;
(v) Falten der Leiterplatte (350), so dass die optischen Köpfe über den offenen Enden der ersten Hülse (305) positioniert sind;
(vi) Platzieren von Kuppeln (302, 302') über den optischen Köpfen;
(vii) Bringen der Kuppeln (302, 302') in Anlage mit der ersten Hülse (305), so dass die erste Hülse (305) und die Kuppeln (302, 302') ein geschlossenes Gehäuse bilden, das die Leiterplatte (350) und die optischen Köpfe umschließt; und
(viii) Verbinden der Kuppeln (302, 302') mit der ersten Hülse (305).

2. Verfahren nach Anspruch 1, mit den folgenden weiteren Schritten:
(a) Platzieren mindestens einer Batterie (345) in einer zweiten Hülse, die zwei entgegengesetzte offene Enden aufweist; und
(b) Platzieren der zweiten Hülse in der ersten Hülse (305).

3. Verfahren nach Anspruch 2, wobei die Schritte (a) und (b) vor dem Schritt (v) ausgeführt werden.

4. Verfahren nach einem der voranstehenden Ansprüche, mit dem weiteren Schritt des Platzierens mindestens einer Batterie (345) in der ersten Hülse (305), wobei der weitere Schritt vor dem Schritt (v) durchgeführt wird.

5. Verfahren nach einem der Ansprüche 1 bis 3, wobei nach dem Positionieren eines der optischen Köpfe über einem der offenen Enden mindestens eine Batterie (345) in der ersten Hülse (305) platziert wird.

6. Verfahren nach einem der voranstehenden Ansprüche, wobei die Kuppeln (302, 302') mit der ersten Hülse (305) durch einen Prozess verbunden werden, der aus der folgenden Gruppe ausgewählt ist: Kleben, Reibpassen, Presspassen, Verrasten bzw. Schnapppassen, Laserschweißen, Laserschmelzen, Reibungsschweißen bzw. Rotationsschweißen und Ultraschallschweißen.

7. Verfahren nach einem der voranstehenden Ansprüche, des Weiteren mit einem Schritt des Einfügens mindestens eines Batteriekontakts (551) in mindestens einen Vorsprung (552), der innerhalb der ersten Hülse (305, 500) bereitgestellt ist.

8. Verfahren nach einem der voranstehenden Ansprüche, wobei der Schritt des Anordnens des Weiteren den Schritt des Führens des flexiblen Abschnitts (322) longitudinal zwischen die entgegengesetzten offenen Enden der ersten Hülse (305) aufweist.

## Revendications

1. Procédé pour l'assemblage d'un dispositif (40) d'imagerie in vivo comprenant les étapes de :
(i) prévoir deux têtes optiques et un circuit imprimé (350) ayant deux parties rigides (351, 361) reliées par une partie flexible (322) ;
(ii) fixer les têtes optiques aux parties rigides (351, 361) ;
(iii) prévoir un premier manchon (305) ayant deux extrémités opposées ouvertes ;
(iv) disposer ladite partie flexible (322) dans une gorge prévue dans ledit premier manchon (305) ;
(v) plier le circuit imprimé (350) de façon que les têtes optiques soient positionnées sur les extrémités ouvertes dudit premier manchon (305) ;
(vi) placer des dômes (302, 302') sur les têtes optiques ;
(vii) disposer les dômes (302, 302') en butée contre le premier manchon (305) de façon que ce dernier et les dômes forment un logement fermé englobant le circuit imprimé (350) et les têtes optiques ; et
(viii) joindre les dômes (302, 302') au premier manchon (305).

2. Procédé selon la revendication 1, **caractérisé en ce qu'**il comporte les étapes supplémentaires de :
(a) placer au moins une batterie (345) dans un second manchon ayant deux extrémités ouvertes opposées ; et
(b) placer le second manchon dans le premier manchon (305).

3. Procédé selon la revendication 2, **caractérisé en ce que** les étapes (a) et (b) sont réalisées avant l'étape (v).

4. Procédé selon l'une des revendications précédentes, comprenant l'étape supplémentaire de placer au moins une batterie (345) dans le premier manchon (305), **caractérisé en ce que** ladite étape additionnelle est réalisée avant l'étape (v).

5. Procédé selon les revendications 1 à 3, **caractérisé en ce qu'**au moins une batterie (345) est placée dans le premier manchon (305) après positionnement d'une des têtes optiques sur l'une des extrémités ouvertes.

6. Procédé selon l'une des revendications précédentes, **caractérisé en ce que** les dômes (302, 302') sont joints au premier manchon (305) par un procédé choisi parmi le groupe suivant : collage, ajustement par friction, ajustement par pression, ajustement par clipsage, soudage par laser, soudage par rotation (spin) et sondage ultrason.

7. Procédé selon l'une des revendications précédentes, **caractérisé en ce qu'**il comporte en outre une étape d'insérer au moins un contact de batterie (551) dans au moins une partie en saillie (552) prévue dans ledit premier manchon (305,500).

8. Procédé selon l'une des revendications précédentes, **caractérisé en ce que** l'étape de localisation comporte en outre le fait de passer la partie flexible (322) longitudinalement entre les extrémités ouvertes opposées dudit premier manchon (305).
